# FASCICULE DE BREVET EUROPEEN

(11) **EP 0 873 750 B1**
(45) Date de publication et mention de la délivrance du brevet: **02.06.2004**
(21) Numéro de dépôt: 98400897.9
(22) Date de dépôt: 10.04.1998
(51) Int. Cl.: A61K 9/00

(54) **Comprimés bioadhésifs**
Bioadhäsive Tabletten
Bioadhesive tablets

(30) Priorité: 23.04.1997 FR 9705028
(43) Date de publication de la demande: 28.10.1998
(73) Titulaire: PERMATEC TECHNOLOGIE AG, 6301 Zug (CH)
(72) Inventeur: Rault, Isabelle, 68100 Mulhouse (FR)
(74) Mandataire: Peaucelle, Chantal

(56) Documents cités:
- EP-A- 0 020 777
- EP-A- 0 262 422
- EP-A- 0 649 650
- WO-A-95/34286
- FR-A- 2 514 642

## Description

L'invention concerne des comprimés possédant des propriétés bio-adhésives, utilisables par voie transmuqueuse.

L'administration par voie transmucosale présente l'avantage au plan métabolique d'éviter la métabolisation importante du principe actif par effet de premier passage hépatique et donc de diminuer les doses administrées en améliorant l'efficacité clinique. Le principe actif actif ne subit pas les différentes dégradations enzymatiques ou chimiques tout au long du tractus gastro-intestinal, de même que les inconvénients liés à la fonction et à la physiologie de l'appareil gastro-intestinal.

De nombreuses formulations ont été proposées pour améliorer diverses propriétés souhaitées pour de tels comprimés, telles que la biodisponibilité du principe actif, en contrôlant sa vitesse de libération et la direction du flux de principe actif, ou l'adhésivité du comprimé sur un tissu ou une muqueuse.

Dans ces formulations, le composé bioadhésif est utilisé fréquemment en mélange avec le principe actif et d'autres excipients tels qu'agents gonflants. Les propriétés respectives de ces produits ne sont donc pas totalement mises à profit. Une partie du composé bioadhésif est en effet emprisonné dans le mélange et ne sert pas à l'adhésion. De même, la totalité du principe actif n'est pas toujours utilisée et le contrôle de sa vitesse de libération peut s'avérer difficile.

Dans d'autres formulations, les choix effectués pour les produits bioadhésifs rendent nécessaire l'utilisation d'adjuvants, tels que la gélatine, pour obtenir l'effet souhaité.

Junginger H.E. et al. (Deutsche Apotheker Zeitung, N°26, 1991, p.1337-1348) décrivent de manière générale plusieurs comprimés bioadhésifs multi-couches.

EP 0020777 divulgue des comprimés bi-couches qui consistent en une couche adhésive et une couche non-adhésive, la séparation en deux couches permettant une manipulation plus facile ainsi qu'un meilleur contrôle de la libération du principe actif dans la cavité buccale.

De manière générale, les comprimés bioadhésifs actuellement disponibles présentent ainsi plusieurs types de limitations : telles qu'une bioadhésion insuffisante, un encombrement trop important, une structure incohérente à l'usage, ou bien encore une mauvaise maîtrise de l'intensité et/ou de l'orientation des flux de principe(s) actif(s).

Les produits bioadhésifs sont difficiles à travailler industriellement car ils posent des problèmes de collage. Ils sont, du fait de leur nature hydrophile, difficiles à travailler en granulation humide ; travaillés en mélange de poudres, ils présentent souvent un mauvais écoulement. Enfin, d'une façon générale, ces produits ne présentent pas une bonne aptitude à la compression.

Les inventeurs ont à présent constaté qu'en choisissant certains types de composés bioadhésifs et d'excipients, il était possible de réaliser un nouveau type de comprimés, avec une succession de couches présentant des fonctions distinctes, et d'opérer par compression directe pour élaborer au moins l'une de ces couches.

L'invention a donc pour but de fournir de nouveaux comprimés optimisés quant à leur adhésivité et la diffusion des principes actifs qu'ils renferment, et donc leur efficacité, et de faible coût.

Elle a notamment pour but de fournir des comprimés utilisables par voie transmuqueuse et administrables le cas échéant par voie orale.

Les comprimés de l'invention sont caractérisés en ce qu'ils se présentent sous la forme d'un composé de tri-couches, élaborés pour une utilisation par voie essentiellement transmuqueuse, dans lesquels la couche interne, comprenant la majorité de la charge totale de principe actif est recouverte:
- sur ses deux faces opposées, respectivement:
   - par une couche bioadhésive, comprenant la charge totale de matériau bioadhésif, cette couche étant directement compressible lors de l'élaboration du comprimé, et étant capable d'adhérer à une muqueuse par imprégnation avec de l'eau ou avec un fluide biologique présent dans l'environnement de la muqueuse et permettant une libération telle que souhaitée du principe actif, et
   - par une couche formant une barrière à la diffusion du principe actif et à la pénétration de l'eau ou du fluide biologique
- et sur les côtés soit par ladite couche bioadhésive, soit par ladite couche barrière
ou bien est recouverte sur ses deux faces par la couches bioadhésive et sur ses côtés par la couche barrière.

On observera que la ou les couches bioadhésives peuvent être comprimées directement, ce qui représente un gain technique par rapport à la préparation des comprimés bioadhésifs jusqu'alors disponibles et permet une fabrication à moindre coût.

De plus, les charges totales de principe actif et de matériau bioadhésif se trouvent respectivement dans des couches distinctes.Chaque couche peut être ainsi élaborée avec la quantité minimale de produit requise pour l'effet souhaité. Il en résulte un avantage économique et un intérêt à la fois sanitaire et de confort pour le patient. Grâce à la concentration du polymère bioadhésif au contact du tissu ou de la muqueuse sur lequel est appliqué le comprimé, on obtient un effet bioadhésif intense et une libération contrôlée et reproductible de principe actif.

L'optimisation de la bioadhésion des comprimés de l'invention permet la réalisation de comprimés qui, par rapport à ceux de l'art antérieur, présentent une efficacité et une durée de bioadhésion accrue pour une quantité équivalente ou inférieure car optimisée de composé bioadhésif. Cet avantage présente un intérêt tout particulier pour une administration de principes actifs sur une longue période.

Le comprimé selon l'invention présente l'avantage non seulement d'orienter la diffusion du principe actif mais aussi de limiter les phénomènes de délitement de la matrice en protégeant notamment le comprimé de l'érosion due aux mouvements des tissus voisins (par exemple de la lèvre supérieure, dans le cas d'un comprimé utilisable par voie buccale) en empêchant un collage inapproprié du comprimé.

Selon un mode de réalisation avantageux, la couche bioadhésive des comprimés de l'invention renferme, en mélange avec le matériau bioadhésif, au moins un agent gonflant et insoluble ou un agent gonflant et peu soluble en présence de fluide biologique et/ou un agent gonflant et soluble ou un agent gélifiable et soluble en présence de fluide biologique, avec le cas échéant au moins un excipient permettant d'améliorer la bioadhésion et/ou un adjuvant soluble dans l'eau ou agissant comme un agent à caractère hydrophile.

De manière avantageuse, le matériau bioadhésif est essentiellement constitué par un polymère modifié par de l'anhydride maléique ou par un dérivé tel qu'acide, ester, ou sel pharmaceutiquement acceptable.

Il s'agit de préférence d'un copolymère de méthylvinyléther et d'anhydride maléique.

Dans le contexte de l'invention, axé sur l'élaboration de structures multicouches, ce polymère est en effet utilisé comme bioadhésif à part entière, sans qu'il soit nécessaire comme dans l'art antérieur de le mélanger à d'autres bioadhésifs et/ou de le soumettre à des traitements particuliers.

Des exemples de copolymères de ce type actuellement disponibles dans le commerce correspondent à ceux commercialisés sous la marque Gantrez ® (GAF products).

Ils comprennent les Gantrez ® AN (forme anhydride), S (forme acide), ES (forme ester) et MS (forme sel sodique et calcique).

Les agents gonflants et insolubles ou les agents gonflants et peu solubles en présence de fluide biologique sont avantageusement choisis parmi le groupe des éthers de cellulose comme la carboxyméthylcellulose sodique, l'hydroxypropylcellulose réticulée l'hydroxypropylcellulose de haut poids moléculaire, le groupe des esters de cellulose entériques et non entériques, les amidons modifiés comme le carboxyméthyl amidon, le copolymère de divinylbenzène/méthacrylate de potassium, le groupe des dérivés de l'acide méthacrylique comme les polyméthylméthacrylates, le groupe des crospovidones/crospolyvidones, l'alcool polyvinylique de haut poids moléculaire, le groupe de l'acide alginique et de ses dérivés, le groupe des dérivés de l'acide acrylique comme l'acide acrylique réticulé avec du divinylglycol et son sel de calcium, le groupe des carraghénanes et de leurs dérivés, le copolymère de vinylacétate et de l'acide crotonique.

Des agents gonflants et solubles ou des agents gélifiables et solubles en présence de fluide biologique convenant pour la mise en oeuvre de l'invention comprennent le groupe des éthers de cellulose comme la méthylcellulose, la carboxyméthylcellulose sodique, l'hydroxypropylméthylcellulose de faible poids moléculaire, le groupe des esters de cellulose entériques et non entériques, l'alcool polyvinylique de faible poids moléculaire, l'alcool polyvinylique de moyenne viscosité, le polyoxyéthylèneglycol, le groupe des povidones/polyvidones/copolyvidones, les scléroglucannes, les amidons et les amidons modifiés comme les amidons prégélatinisés, le groupe des carraghénanes et de leurs dérivés, le groupe de l'acide alginique et de ses dérivés.

D'autres excipients, utilisés en association avec le matériau bioadhésif, peuvent améliorer la bioadhésion comme la gomme guar, la gomme xanthane, le caroube, les carraghénates, la pectine, une protéine biologique ou synthétique utilisée seule ou en association avec d'autres protéines d'origine biologique ou synthétique, les cyclodextrines ou dérivés tels que les bétacyclodextrines, les hydroxypropylbétacyclodextrines, les bétacyclodextrines partiellement méthylées, les dérivés de l'acide acrylique, comme l'acide acrylique réticulé avec du divinylglycol et son sel de calcium.

Des adjuvants solubles dans l'eau ou agissant comme des agents à caractère hydrophile sont choisis parmi le lactose, le mannitol, la silice colloïdale, les excipients du groupe des hydrocelluloses comme la cellulose microcristalline, les excipients dans le groupe des éthers de cellulose, la gélatine, les polyéthylèneglycols (PEG), les poloxamers et les pyrrolidones.

La couche bioadhésive renferme avantageusement 5 à 100 % de matériau bioadhésif, de 0 à 80% d'agent gonflant et insoluble ou d'agent gonflant et peu soluble, de 0 à 50 % d'agent gonflants et solubles ou d'agent gélifiable et soluble, de 0 à 50 % d'excipient qui utilisé en association avec le polymère bioadhésif peut améliorer la bioadhésion et de 0 à 80 % d'agent adjuvant soluble dans l'eau agissant comme un agent à caractère hydrophile.

Comme déjà souligné, l'élaboration d'une telle couche adhésive permet de limiter le phénomène de "collage" du comprimé sur un tissu autre que celui sur lequel on souhaite l'appliquer. En effet, la présence du polymère bioadhésif est limitée à la surface en contact avec la muqueuse ou le tissu. Dans le cas d'un comprimé buccoadhésif, la présence d'une couche adhésive facilite nettement la pose du comprimé, limite ses déplacements sur la gencive et sur la lèvre pendant les premiers temps de l'application, ce qui améliore la bioadhésion et enfin augmente le confort du patient. Toutes ces améliorations font que le risque jusque là présent de l'ingestion par voie orale du comprimé buccoadhésif, dû à une mauvaise bioadhésion durant les premiers temps de l'application, est très fortement diminué.

Dans la couche adhésive, on peut également ajouter des corps gras pour des raisons de technologie pharmaceutique dans des proportions allant de 0 à 50 % de la masse totale de la couche. Il est clair que ce type d'excipient à caractère hydrophobe est susceptible d'intervenir sur les profils de diffusion du principe actif à travers la couche adhésive.

En ce qui concerne la couche interne avec la majorité de la charge de principe actif, appelée également ci-après couche principale elle renferme avantageusement, en mélange avec le ou les principes actifs, au moins un agent gonflant et insoluble ou un agent gonflant et peu soluble en présence de fluide biologique et/ou au moins un agent gonflant et soluble ou un agent gélifiable et soluble en présence de fluide biologique, avec le cas échéant au moins un excipient permettant de réaliser des inclusions de principe actif et/ou au moins un excipient soluble dans l'eau ou agissant comme un agent à caractère hydrophile, ou au moins un excipient insoluble dans l'eau ou agissant comme un agent à caractère hydrophobe.

Les agents déjà énumérés en rapport avec la couche bioadhésive sont choisis de préférence parmi les produits respectivement indiqués ci-dessus pour chaque type d'agents.

Les excipients permettant de réaliser des inclusions de principe actif comprennent par exemple les cyclodextrines ou dérivés tel que les bétacyclodextrines, les hydroxypropylbétacyclodextrines, les bétacyclodextrines partiellement méthylées et les glycérides comme le monooléate de glycéryle.

Les adjuvants insolubles dans l'eau ou agissant comme des agents à caractère hydrophobe sont notamment choisis parmi l'huile de ricin hydrogénée, le stéarate de magnésium, les huiles naturelles et synthétiques, les cires naturelles ou semi-synthétiques, les esters d'acides gras et de polyoxyéthylène, les acides gras et les esters d'acides gras (mono et triglycérides) et leurs dérivés comme les acides gras polyéthoxylés (PEG-stéarate,...), les alcools gras et esters d'alcool gras et leurs dérivés comme les alcools gras polyéthoxylés (octyldodeceth-25,...) et le polychlorure de vinyle.

Le ou les principes actifs sont notamment choisis parmi un antihistaminique, un anticholergénique, un élément minéral, un allergène, un anesthésiant de surface , local ou général, un antipyrétique, un antalgique non opiacé,un antalgique opiacé, un antispasmodique anticholinergique et non anticholinergique, un antiinflammatoire non stéroïdien tels que l'acide taiprofénique, l'indométhacine, le diclofénac, l'ibuprofène, le kétoprofène, le naproxène, le piroxicam, un antiinflammatoire stéroïdien comme la béthmétasone, la prédnisolone, un cytotoxique, un agent antihormonal, un antianémique, un antiémétique, un antiasthénique, un antihypertenseur et parmi eux les béta-bloquants comme le propranolol, l'aténolol, le métoprolol, les inhibiteurs de l'enzyme de conversion comme le captopril, l'énalapril, les antagonistes de l'angiotensine II, les inhibiteurs calciques tels que la nifédipine et le diltiazem, les antihypertenseurs d'action centrale, les vasodilatateurs, un hypolipémiant, un antidiabétique oral, un anticoagulant, un antiaggrégant plaquettaire, un inhibiteur calcique, un dérivé nitré utilisé dans le traitement de l'insuffisance coronarienne, un antiangoreux non nitré, un diurétique, un dérivé de la digitaline et apparenté, un antiarythmique, un antihypotenseur et analeptique circulatoire, un vasodilatateur, un anti-ischémique, un vasculoprotecteur et un veinotonique, une hormone, un antiherpétique, un antiphotosensibilisant, un antiulcéreux comme la ranitidine, la cimétidine, un antiacide, un laxatif, un antidiarrhéique, un antifongique, un cholélitholytique, un interféron, un enzyme, un antispasmodique,un antibactérien, un antiseptique, un antiherpétique, un utérorelaxant, un ocytocique, un estrogène, un progestratif, un estroprogestatif, un principe actif indiqué dans la lactation comme la bromocriptine, un principe actif indiqué dans le traitement de la stérilité, un antigonadotrope, un anticoagulant, un thrombolytique, un antifibrinolytique, une vitamine, un hémostatique, une cyclosporine, un agent alkylant, un antibiotique, un antiviral, un antiparasitaire, un vaccin, un produit de diagnostic, un principe actif indiqué dans le traitement de l'obésité, un oréxigène, un principe actif indiqué dans le traitement des corrections des anomalies métaboliques, un principe actif indiqué dans la nutrition orale et entérale, un anticonvulsivant, un antiparkinsonien, un antimyasthénique, un principe actif indiqué dans le traitement de la maladie d'Alzheimer, un antimigraineux, un neuroleptique, un anxiolytique, un hypnotique, un sédatif, un antidépresseur, un normothymique, un psychostimulant, un principe actif indiqué dans le traitement des états de dépendance en alcoollogie, en désintoxication tabagique, en désintoxication des opiacées, un antiglaucomateux, un mydriatique, un brochodilatateur, un antiasthmatique, un antitussif, un fluidifant bronchique, un révulsif (topique), un principe actif indiqué dans le traitement des ostéopathies, un principe actif indiqué dans le traitement dans l'accés aigu de goutte, un principe actif indiqué dans le traitement hypo-uricémiant, un principe actif indiqué dans le traitement des algodystrophies, un myorelaxant, un principe actif indiqué dans le traitement de l'arthrose, un correcteur des hypposialies, un principe actif indiqué dans le traitement de la lithiase urinaire, un principe actif indiqué dans le traitement de l'insuffisance rénale, un principe actif indiqué dans le traitement de l'énurésie, un principe actif indiqué dans le traitement de l'éjaculation rétrograde, un principe actif indiqué dans le traitement de l'impuissance, et autre.

Parmi les molécules entrant dans la composition selon l'invention, on peut citer à titre non limitatif, les agents de contraste, les radioéléments, les minéraux et les colorants.

La couche principale renferme avantageusement de 70 à 100% de la charge totale du comprimé en principe actif, de 0 à 50% d'agent gonflant et insoluble ou d'agent gonflant et peu soluble, de 0 à 50 % d'agent gonflants et solubles ou d'agent gélifiable et soluble, de 0 à 50 % d'excipient permettant de réaliser des inclusions de principe actif, de 0 à 50 % d'agent adjuvant soluble dans l'eau agissant comme un agent à caractère hydrophile, de 0 à 50 % d'agent adjuvant insoluble dans l'eau agissant comme un agent à caractère hydrophobe.

Comme indiqué plus haut, les comprimés tri-couches selon l'invention comprennent au moins une couche barrière.

La ou les couches barrières sont essentiellement formées. du principe actif dans une proportion pouvant aller de 0 à 30 % de la charge totale d'au moins un agent gonflant et insoluble ou un agent gonflant et peu soluble en présence de fluide biologique et/ou un agent gonflant et soluble ou un agent gélifiable et soluble en présence de fluide biologique, avec le cas échéant au moins un excipient permettant de réaliser des inclusions de principe actif, et/ou un adjuvant soluble dans l'eau ou agissant comme un agent à caractère hydrophile et/ou au moins un agent insoluble dans l'eau ou agissant comme un agent à caractère hydrophobe.

Ces différents agents sont avantageusement choisis parmi les composés donnés ci-dessus en rapport avec chaque type.
La couche barrière renferme avantageusement de 0 à 30 % de la charge totale du comprimé en principe actif, de 0 à 80% d'agent gonflant et insoluble ou d'agent gonflant et peu soluble, de 0 à 80 % d'agent gonflants et solubles ou d'agent gélifiable et soluble, de 0 à 50 % d'excipient permettant de réaliser des inclusions de principe actif, de 0 à 25 % d'agent adjuvant soluble dans l'eau agissant comme un agent à caractère hydrophile, de 0 à 50 % d'agent adjuvant insoluble dans l'eau agissant comme un agent à caractère hydrophobe.

Les trois types de couches ci-dessus comprennent également le cas échéant, d'autres excipients ou substances choisis parmi ceux jouant les rôles :
- de diluants, comme le lactose et ses dérivés ou le dihydrogénophosphate dicalcique, la cellulose microcristalline et ses produits composés comme l'association cellulose microcristalline et silice colloïdale (Prosolv SMCC de mendell), les amidons et leurs dérivés comme les amidons prégélatinisés,
- de liants comme la povidone et la polyvidone, l'éthylcellulose, l'acide alginique et ses dérivés, les maltodextrines, le lactose monohydrate cristallin, anhydre ou modifié ou entrant dans un produit composé comme des composés formés de lactose et de cellulose microcristalline, la cellulose microcristalline et ses produits composés comme l'association cellulose microcristalline et silice colloïdale (Prosolv SMCC de mendell), l'hydroxypropylcellulose partiellement substituée, les amidons et leurs dérivés comme les amidons prégélatinisés, le talc,
- de lubrifiants, comme le stéarate de magnésium, les esters d'alcool gras, les esters d'acides gras, le stéaryl fumarate de sodium, les huiles végétales hydrogénées, les dérivés du polyéthylèneglycol,
- d'agents d'écoulement, comme la silice colloïdale, les amidons modifiés comme l'amidon prégélatinisé,
- des agents de masquage de goût tels que certains dérivés de l'acide méthacrylique,
- dans le maintien de la cohésion et de l'aspect général du comprimé, c'est-à-dire des excipients ou substances permettant de suivre l'évolution du comprimé in situ de façon à éviter, par exemple, des phénomènes de clivages, la formation de trous, des phénomènes de gonflement non homogène entre les différentes couches du comprimé.

On peut également leur incorporer
- des promoteurs d'absorption,
- des agents de solubilisation comme les polysorbates, les pyrrolidones, les dérivés de polyéthylèneglycols, les dérivés de propylène glycol et du glycérol, les dérivés du polyéthylèneglycol comme l'hydroxystéarate de glycérol-polyéthylèneglycol,
- d'aromatisants,
- de colorants,
- d'édulcorants,
- de plastifiants,
- d'antioxydants,
- d'agents de délitement ou de désagrégation comme la croscarmellose sodique, le carboxyméthylamidon sodique, l'hydroxypropylcellulose partiellement substituée, la crospovidone, le glycolate d'amidon sodique,
- d'agents de pelliculage, d'enrobage et les agents filmogènes comme l'hydroxypropylcellulose, les dérivés de l'acide méthacrylique, le copolymère d'acétate de vinyle-acide crotonique,
- d'agents entrant dans la composition solution de polissage et brillantage,
- les agents assurant une protection thermique du principe actif comme les dérivés de saccharose,

Les différentes couches composant le comprimé peuvent contenir des minigranules ou des microcapsules ou des nanoparticules ou des nanogranules ou des nanocapsules enrobées ou non.

On mesurera que les comprimés de l'invention peuvent présenter des structures particulières (disposition relative des couches) permettant d'orienter le flux de principes actifs vers la ou les voies d'administration désirée(s).

Le (ou les) composé(s) présent(s) dans la couche adhésive (en dehors du composé bioadhésif proprement dit) peuvent être identiques ou différents du ou des composé(s) présents dans la couche principale du comprimé. Le (ou les) composés de la couche adhésive sont préférentiellement une ou des hydroxypropylméthylcelluloses (HPMC) de viscosité moyenne ou de haute viscosité tels que les Méthocel K 15M et K 100M P (COLORCON). De manière avantageuse, cet excipient intervient dans le phénomène de bioadhésion, car il permet l'établissement d'un hydrogel dont le gonflement est progressif, ce qui favorise la bioadhésion à long terme.

Si on désire obtenir une adhésion à court terme, on choisit une hydroxypropylméthylcellulose de faible viscosité ou un amidon de maïs ou tout autre excipient parmi les composés cités dans la composition de la couche adhésive.

Dans le cas où l'on désire obtenir une libération progressive et constante du principe actif, on choisit de façon préférentielle le (ou les) même(s) excipient(s) assurant une libération contrôlée, pour la couche principale et la couche adhésive du comprimé. Ceci, dans la mesure où le composé choisi permet d'obtenir, lorsqu'il est associé au polymère bioadhésif, une bioadhésion correspondant au temps d'application souhaité.

Dans le cas où le but est l'obtention d'un profil de libération rapide du principe actif (pic plasmatique), suivie d'une libération prolongée et constante du principe actif, le ou les excipients non bioadhésifs présents dans la couche adhésive sont choisis pour leur faculté à former rapidement un hydrogel et leur compatibilité avec le polymère bioadhésif. Le ou les composés non bioadhésifs composant la couche principale du comprimé sont choisis de manière à favoriser la libération du principe actif par voie orale par un ou des phénomènes de diffusion favorisée (porosité), de délitement et autres.

Les profils de libération rapide ou lent sont également obtenus en associant ou non à la description ci-dessus une technologie pharmaceutique particulière de fabrication des couches.

Les profils de libération rapide sont obtenus préférentiellement par compression directe et les profils lents par un procédé de granulation humide de la couche principale et/oui de la couche barrière.

Des dimensions particulièrement appropriées compte tenu des applications envisagées correspondent à environ 2 à 30 mm de largeur et 1 à 5 mm d'épaisseur environ.

De manière générale, les comprimés tels que définis ci-dessus peuvent présenter un format plat, hémi-convexe, oblong, parallépipédique, chainfréné ou non ou toute forme géométrique présentant l'intérêt d'assurer ile meilleur maintien prolongé du comprimé sur son site d'action. Ces comprimés peuvent être sécables. Ils peuvent être percés.

Les comprimés peuvent renfermer des minigranules, des microcapsules, des nanoparticules ou des nanocapsules enrobées ou non.

Les nouveaux comprimés selon l'invention trouvent des applications médicales, thérapeutiques ou à titre de prévention, ou hygiéniques, chez l'homme ou l'animal. Ils peuvent être utilisés avec avantage pour une administration, à travers la muqueuse buccale (jugale ou gingivale) vaginale, anale, nasale, rectale ou dans le tractus gastro-intestinal ou sur un organe interne par chirurgie. Selon leur structure, la libération de principe actif s'effectue ou non par voie systémique. On notera que les formes galéniques de petite dimension sont particulièrement appropriées pour des applications en opthamologie, sur la conjonctive de l'oeil ,notamment le cul-de-sac conjonctival, et la cornée.

Le comprimé peut être également employé en vertu de son caractère bioadhésif en tant que support bioadhésif intégré dans un processus thérapeutique ou hygiénique. Par exemple, le comprimé peut être chargé en composé permettant d'établir un champ magéntique localisé (grâce à la bioadhésion). Ce champ magnétique lorsqu'il est activé permet le fonctionnement d'une seconde forme gaénique et la délivrance localisée en un lieu et en un temps de substance.

L'invention vise également un procédé de fabrication des comprimés définis ci-dessus.

Le procédé est caractérisé en ce qu'il comprend :
- la compression directe du mélange d'ingrédients élaboré pour former la couche bioadhésive,
- la compression directe du mélange d'ingrédients élaboré pour constituer la couche de principe actif et de celui élaboré pour constituer la couche barrière, ou la compression de l'un de ces mélanges ou des deux mélanges tels qu'obtenus par granulation par voie humide ou par granulation sèche.

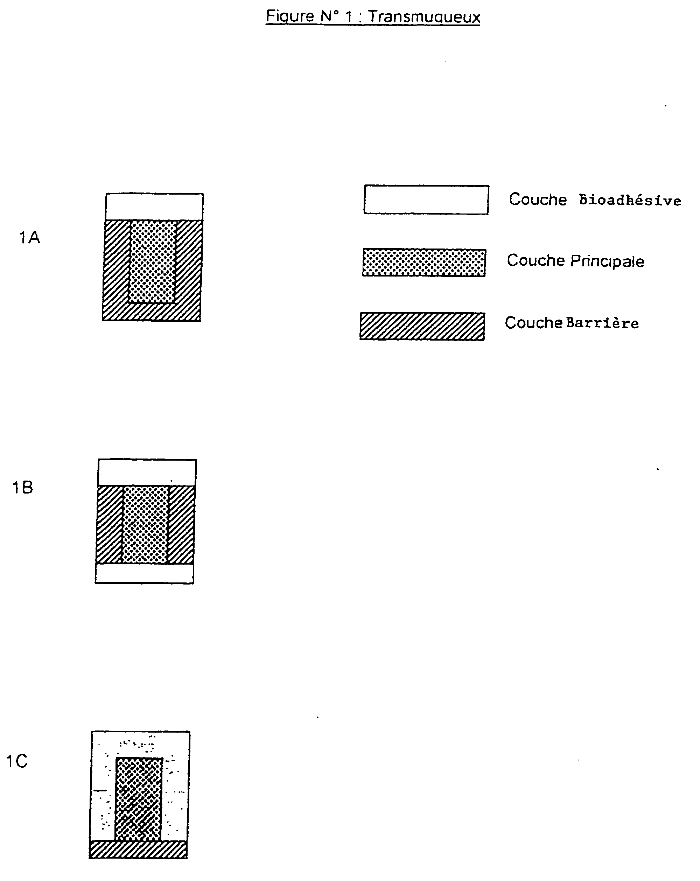

D'autres caractéristiques et avantagés de l'invention seront donnés dans 1 exemple qui suit, avec référence aux figures 1A à 1C, qui représentent des dispositions relatives de couches qui peuvent être adaptées à une voie essentiellement transmuqueuse.

Pour chacune de ces figures, la légende correspondante est :

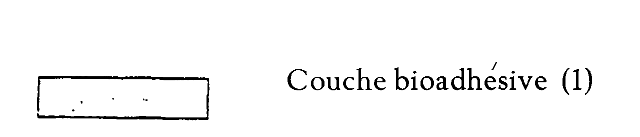

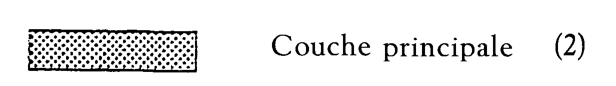

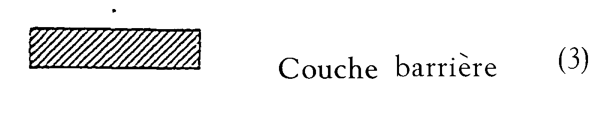

### Exemple 1

### a) Préparation des mélanges utilisés pour former les différentes couches

- mélange A utilisé pour former la couche avec le principe actif ou en couche principale :
On pèse les excipients suivants pour fabriquer l'équivalent de 100 couches principales.

| Excipients | Quantités |
|---|---|
| Mélatonine (Société Sigma) | 0,3 g |
| Ethylcellulose (Ethylcellulose NF 100 - SociétéAqualon) | 0,5 g |
| dihydrogéno Phosphate dicalcique (Encompress - Société SPCI) | 6,1 g |
| Hydroxypropylméthylcellulose (Méthocel K100M - Société Colorcon) | 3,0 g |
| Stéarate de magnésium (Société Lambert Rivière) | 0,1 g |

La mélatonine, le phosphate dicalcique et la moitié de la quantité d'éthylcellulose sont mélangés au Turbula pendant 10 minutes. Le mélange est passé au travers d'un tamis ayant une ouverture de maille de 0,8 mm. Le reste de l'éthylcellulose est dissous dans 5 ml d'éthanol. On granule le mélange avec cette solution alcoolique d'éthylcellulose au Kitchen Aid à vitesse 2 pendant 10 minutes. La masse obtenue est calibrée sur un tamis d'ouverture de maille de 0,8 mm. Après séchage, les grains sont tamisés (ouverture de maille: 0,4 mm). On mélange l'hydroxypropylméthylcellulose et les grains obtenus. On ajoute ensuite le stéarate de magnésium, puis on homogénéise le mélange.
- mélange B utilisé pour former couche bioadhésive :
On pèse les excipients suivants pour fabriquer l'équivalent de 100 couches bioadhésives.

| Excipients | Quantités |
|---|---|
| Copolymère de méthylvinyléther et d'anhydride maléique (Gantrez MS 955 - Société ISP) | 4,87 g |
| Hydroxypropylméthylcellulose (Méthocel K100M - Société Colorcon) | 4,87 g |
| Stéarate de magnésium (Société Lambert Rivière) | 0,1 g |
| Silice colloïdale (Société Lambert Rivière) | 0,06 g |
| Oxyde de fer jaune (Sicovit) (Société BASF) | 0,1 g |

Les produits peuvent être mélangés directement avant compression.
Le copolymère de méthylvinyléther et d'anhydride maléique est mélangé à l'hydroxypropylméthylcellulose dans un Turbula pendant 10 minutes. On ajoute le stéarate de magnésium, la sillice colloïdale et l'oxyde de fer jaune, et on mélange l'ensemble au Turbula pendant 4 minutes.
- mélange C utilisé pour former la couche barrière:
On pèse les excipients suivants pour fabriquer l'équivalent de 100 couches barrières.

| Excipients | Quantités |
|---|---|
| Ethylcellulose (Ethylcellulose NF 100 - Société Aqualon) | 0,9 g |
| Dihydrogénophosphate dicalcique (Encompress - Société SPCI) | 6,9 g |
| Hydroxypropylméthylcellulose (Méthocel K100M - Société Colorcon) | 2,0 g |
| Stéarate de magnésium (Société Lambert Rivière) | 0,1 g |
| Oxyde de fer rouge (Société BASF) | 0,1 g |

La quantité d'éthylcellulose est divisée en deux fractions égales. Une première fraction est mélangée au Turbula pendant 10 minutes à la totalité de l'hydrogénophosphate dicalcique. Le mélange obtenu est tamisé sur 0,8 mm d'ouverture de maille. Le reste de l'éthylcellulose est dissous dans 5 ml d'éthanol. On granule le mélange précédent avec cette solution alcoolique d'éthylcellulose dans un Kitchen Aid à vitesse 2 pendant 10 minutes. La masse est calibrée sur un tamis d'ouverture de maille de 0,8 mm. Après séchage, les grains sont tamisés (ouverture de maille: 0,4 mm). On mélange l'hydroxypropylméthylcellulose avec les grains. On ajoute ensuite le stéarate de magnésium et l'oxyde de fer rouge, puis on homogénéise le mélange au Turbula pendant 4 minutes.

### b) étape de compression

Les compressions sont réalisées sur une machine à comprimer alternative KORSH de type EKO de façon manuelle de manière à obtenir des comprimés plats, de forme ronde. L'épaisseur des comprimés est de 2 mm et leur diamètre est de 10 mm.

On réalise la compression des 100 mg de mélange A correspondant à la couche principale dans une matrice avec un format plat de diamètre 8 mm. Les comprimées obtenus sont récupérés.
On remplit la matrice de format 10 mm avec 50 mg de mélange C, on comprime légèrement.
On pose le comprimé de format 8 mm de diamètre dans la chambre de compression de façon centrée. On remplit la chambre avec les 50 mg restants de mélange C. On arase jusqu'au niveau supérieur du comprimé. On comprime légèrement.
On procède ensuite à l'alimentation de la chambre de compression avec 100 mg de mélange B. On arase et on réalise la compression finale.
On obtient un comprimé plat terminé à 300 mg. Il présente la structure illustrée par la figure 1A avec une couche bioadhésive (1) et une couche principale (2) recouverte sur ses côtés et sa surface par une couche barrière (3)

Il est également possible en travaillant avec le mélange B en première et en dernière étape et en plaçant du mélange C uniquement sur les côtés selon la technique détaillée ci-dessus de réaliser la structure illustrée par la figure 1B, qui comprend deux couches bioadhésives (1) externes emprisonnant une couche de principe actif (2) dont les côtés sont recouverts de couche barrière (3).

En variante, on élabore un comprimé tel que représenté sur la figure 1C en remplissant la chambre de compression avec le 50 mg de mélange B, que l'on compresse, puis en plaçant au centre de la matrice le comprimé préalablement réalisé avec le mélange A puis en ajoutant autour du comprimé 50 mg de mélange B que l'on comprime pour former la couche bioadhésive (1). On introduit ensuite dans la trémie le mélange C pour constituer la couche barrière (3).

Le comprimé peut être placé sur la muqueuse gingivale d'un patient et pourra délivrer de la mélatonine de manière contrôlée pendant 8 heures sans se déliter, sans se décoller et sans se coller de manière inappropriée. Un tel design permet une libération de mélatonine par voie essentiellement transmuqueuse et une utilisation en quantité réduite de principe actif et d'excipients.

### Exemple 2

### a) préparation des mélanges A, B et C

On prépare les mélanges A et B en procédant comme dans l'exemple 1, mais en utilisant pour le mélange A, les excipients suivants, selon les quantités indiquées qui permettent de fabriquer l'équivalent de 100 couches principales.

| Excipient | Quantités |
|---|---|
| Prochlorpérazine | 0,5 g |
| (Société RPR) | |
| Ethylcellulose | 0,25 g |
| (Ethylcellulose NF 100 - | |
| Société Aqualon) | |
| Dihydrogénophosphate dicalcique | 2,7 g |
| (Encompress - Société SPCI) | |
| Hydroxypropylméthylcellulose | 1,5 g |
| (Méthocel K100M - Société Colorcon) | |
| Stéarate de magnésium | 0,05 g |
| (Société Lambert Rivière) | |

Pour préparer le mélange C, on utilise les excipients suivants pour fabriquer l'équivalent de 100 couches barrière.

| Excipients | Quantités |
|---|---|
| Hydroxypropylcellulose (Klucel - Société Aqualon) | 1,0 g |
| Dihydrogénophosphate dicalcique (Encompress - Société SPCI) | 2,8 g |
| Polyvinylpyrrolidone (Plasdone K29-32 - Société ISP) | 0,1 g |
| Hydroxypropylméthylcellulose (Méthocel K100M - Société Colorcon) | 1,0 g |
| Stéarate de magnésium (Société Lambert Rivière) | 0,05 g |
| Oxyde de fer rouge (Sicovit - société BASF) | 0,05 g |

L'hydroxypropylcellulose est mélangée au phosphate dicalcique dans un Turbula pendant 10 minutes. Le mélange obtenu est tamisé sur 0,8 mm. La totalité de la polyvinylpyrrolidone est dissoute dans 3,5 ml d'eau. On granule le mélange précédent avec cette solution dans un Kitechen Aid à vitesse 2 pendant 8 minutes. La masse est calibrée sur un tamis d'ouverture de maille de 0,8 mm. Après séchage, les grains sont tamisés sur 0,4 mm. On mélange l'hydroxypropylméthylcellulose avec les grains dans u Turbula pendant 10 minutes, puis on ajoute le stéarate de magnésium et l'oxyde de fer rouge et on homogénéise le mélange au Turbula pendant 4 minutes.

### b) étape de compression

Les compressions sont réalisées sur une machine à comprimer alternative KORSH de type EKO de manière à obtenir des comprimés plats de forme ronde.

Un comprimé de format plat et rond et de diamètre 6 mm est réalisé à partir du mélange A (couche principale).

On remplit la chambre de compression avec 50 mg de mélange C. On pré-comprime (couche barrière).On dépose le comprimé réalisé avec le mélange A au dessus de la couche barrière, au centre de la matrice. On remplit ensuite avec 50 mg de mélange B de manière à recouvrir le comprimé formant la couche principale contenant le principe actif (prochlorpérazine). Une compression finale est réalisée.

On obtient un comprimé plat de forme ronde de diamètre 8 mm, pesant 150 mg environ, qui présente le design illustré par la figure 1C.
Le comprimé peut être placé à l'intérieur d'une cavité d'une muqueuse buccale d'un patient atteint de nausées et vomissements. Il pourra délivrer de la Prochlorpérazine de manière contrôlée pendant 5 heures sans se détériorer. Un tel design permet une libération prolongée, essentiellement par voie transmuqueuse.

### Exemple 3

### a) préparation des mélanges A, B et C

On prépare les mélanges A et B en procédant comme dans l'exemple 1, mais en utilisant pour le mélange A, les excipients suivants, selon les quantités indiquées qui permettent de fabriquer l'équivalent de 100 couches principales.

| Excipients | Quantités |
|---|---|
| Nifédipine (Société SIGMA) | 0,8 g |
| Cellulose microcristalline (Avicel PH 302 - Société SEPPIC) | 0,2 g |
| Lactose Fast Flo (Société SEPPIC) | 1,975 g |
| Hydroxypropylméthylcellulose (Méthocel K4M - Société Colorcon) | 0,5 g |
| Hydroxypropylméthylcellulose (Méthocel K100M - Société Colorcon) | 1,5 g |
| Stéarate de magnésium (Société Lambert Rivière) | 0,025 g |

Les excipients, excepté le stéarate de magnésium, sont mélangés au Turbula pendant 10 minutes. On tamise le mélange obtenu sur 0,8 mm. On ajoute ensuite le stéarate de magnésium puis on mélange au Turbula pendant 4 minutes.

Pour préparer le mélange B :
On pèse les excipients suivants pour fabriquer l'équivalent de 100 couches bioadhésives.

| Excipients | Quantités |
|---|---|
| Copolymère de méthylvinyléther et | 2,435 g |
| d'anhydride maléique | |
| (Gantrez MS 955 - Société ISP) | |
| Hydroxypropylméthylcellulose | 2,435g |
| (Méthocel K100M - Société Colorcon) | |
| Stéarate de magnésium | 0,05g |
| (Société Lambert Rivière) | |
| Silice colloïdale | 0,03g |
| (Société Lambert Rivière) | |
| Oxyde de fer jaune (Sicovit) | 0,05g |
| (Société BASF) | |

Pour préparer le mélange C, on utilise les excipients suivants pour fabriquer l'équivalent de 100 couches barrière.

| Excipients | Quantités |
|---|---|
| Monostéarate de glycérol | 1,0 g |
| (Myvaplex 600 - Société Unipex) | |
| Hydroxypropylméthylcellulose | 3,95 g |
| (Méthocel K100M - Colorcon) | |
| Oxyde de fer rouge | 0,05 g |

Le monostéarate de glycérol est mélangé à l'hydroxypropylcellulose au Turbula pendant 5 minutes. La masse est calibrée sur un tamis d'ouverture de maille de 0,8 mm. On ajoute l'oxyde de fer rouge, on mélange au Turbula pendant 4 minutes.

### b) étape de compression

Les compressions sont réalisées sur une machine à comprimer alternative KORSH de type EKO de manière à obtenir des comprimés plats de forme ronde.

Un comprimé de format plat et rond et de diamètre 6 mm est réalisé à partir du mélange A (couche principale).

On remplit la chambre de compression avec 50 mg de mélange C. On pré-comprime (couche barrière).On dépose le comprimé réalisé avec le mélange A au dessus de la couche barrière, au centre de la matrice. On remplit ensuite avec 50 mg de mélange B de manière à recouvrir le comprimé formant la couche principale contenant le principe actif (nifédipine). Une compression finale est réalisée.

On obtient un comprimé plat de forme ronde pesant 150 mg environ, qui présente le design illustré par la figure 1C.

Le comprimé peut être placé sur la muqueuse buccale d'un patient dans le traitement de la crise hypertensive en remplacement du traitement par voie sublinguale où il assurera la délivrance de la trinitine de manière contrôlée pendant 2 heures sans se détériorer. Un tel design permet une libération rapide de principe actif transmuqueuse.

L'invention fournit ainsi des comprimés bioadhésifs de grande efficacité et présentant une souplesse d'application optimale.

Ils présentent notamment de très faibles risques de décollement accidentel et des surdoses qui peuvent y être associées. Ils ont, l'avantage de maintenir l'adhésion entre les différentes couches et de présenter une répartition homogène des contraintes qui résultent des changements de niveaux d'hydratation des différentes couches. Ils ne présentent ainsi pas les inconvénients liés au clivage de couches tels que libération prématurée ou excessive de principes actifs.

En outre, ils ne provoquent ni gène due à l'encombrement, ni allergies, ni sensibilisations, ni irritations (les effets du ou des principe(s) actif(s) seuls n'étant bien entendu pas considéré(s).

## Revendications

1. Comprimés bioadhésifs, **caractérisés en ce qu'**ils se présentent sous la forme d'un composé de tri-couches, élaborés pour une utilisation par voie essentiellement transmuqueuse, dans lesquels la couche interne, comprenant la majorité de la charge totale de principe actif est recouverte:
- sur ses deux faces opposées, respectivement:
- par une couche bioadhésive, comprenant la charge totale de matériau bioadhésif et ne comprenant pas de principe actif, cette couche étant directement compressible lors de l'élaboration du comprimé, et étant capable d'adhérer à une muqueuse par imprégnation avec de l'eau ou avec un fluide biologique présent dans l'environnement de la muqueuse et permettant une libération telle que souhaitée du principe actif, et
- par une couche formant une barrière à la diffusion du principe actif et à la pénétration de l'eau ou du fluide biologique
- et sur les côtés soit par ladite couche bioadhésive, soit par ladite couche barrière
ou bien est recouverte sur ses deux faces par la couches bioadhésive et sur ses côtés par la couche barrière.

2. Comprimés selon la revendication 1, **caractérisés en ce que** la couche bioadhésive renferme, en mélange avec le matériau bioadhésif, au moins un agent gonflant et insoluble ou un agent gonflant et peu soluble en présence de fluide biologique, et/ou un agent gonflant et soluble ou un agent gélifiable et soluble en présence de fluide biologique, avec le cas échéant au moins un excipient permettant d'améliorer la bioadhésion et/ou un adjuvant soluble dans l'eau ou agissant comme un agent à caractère hydrophile.

3. Comprimés selon l'une quelconque des revendications précédentes, **caractérisés en ce que** le matériau bioadhésif est essentiellement constitué par un polymère modifié par de l'anhydride maléique ou par un dérivé tel qu'acide , ester, ou sel pharmaceutiquement acceptable.

4. Comprimés selon la revendication 3,**caractérisés en ce que** le matériau bioadhésif est essentiellement constitué par un copolymère de méthylvinyléther et d'anhydride maléique.

5. Comprimés selon la revendication 2 ou 4, **caractérisés en ce que** la couche bioadhésive renferme avantageusement 5 à 100 % de matériau bioadhésif, de 0 à 80 % d'agent gonflant et insoluble ou d'agent gonflant et peu soluble, de 0 à 50 % d'agent gonflant et soluble ou d'agent gélifiable et soluble, de 0 à 50 % d'excipient qui, utilisé en association avec le polymère bioadhésif, peut améliorer la bioadhésion, et de 0 à 80 % d'agent adjuvant soluble dans l'eau agissant comme un agent à caractère hydrophile.

6. Comprimés selon l'une quelconque des revendications précédentes, **caractérisé en ce que** la couche principale renferme avantageusement, en mélange avec le ou les principes actifs, au moins un agent gonflant et insoluble ou un agent gonflant et peu soluble en présence de fluide biologique, et/ou au moins un agent gonflant et soluble ou un agent gélifiable et soluble en présence de fluide biologique, avec le cas échéant au moins un excipient permettant de réaliser des inclusions de principe actif et/ou au moins un excipient soluble dans l'eau ou agissant comme un agent à caractère hydrophile, ou au moins un excipient insoluble dans l'eau ou agissant comme un agent à caractère hydrophobe.

7. Comprimés selon la revendication 6, **caractérisés en ce que** la couche principale renferme avantageusement de 70 à 100 % de la charge totale du comprimé en principe actif, de 0 à 50% d'agent gonflant et insoluble ou d'agent gonflant et peu soluble, de 0 à 50 % d'agent gonflant et soluble ou d'agent gélifiable et soluble, de 0 à 50 % d'excipient permettant de réaliser des inclusions de principe actif, de 0 à 50 % d'agent adjuvant soluble dans l'eau agissant comme un agent à caractère hydrophile, de 0 à 50 % d'agent adjuvant insoluble dans l'eau agissant comme un agent à caractère hydrophobe.

8. Comprimés selon l'une quelconque des revendications 1 à 7, **caractérisés en ce que** la couche barrière est essentiellement formée du principe actif dans une proportion pouvant aller de 0 à 30 % de la charge totale, d'au moins un agent gonflant et insoluble ou un agent gonflant et peu soluble en présence de fluide biologique et/ou un agent gonflant et soluble ou un agent gélifiable et soluble en présence de fluide biologique, avec le cas échéant au moins un excipient permettant de réaliser des inclusions de principe actif, et/ou un adjuvant soluble dans l'eau ou agissant comme un agent à caractère hydrophile et/ou au moins un agent insoluble dans l'eau ou agissant comme un agent à caractère hydrophobe.

9. Comprimé selon la revendication 8, **caractérisé en ce que** la couche barrière renferme avantageusement de 0 à 30 % de la charge totale du comprimé en principe actif, de 0 à 80% d'agent gonflant et insoluble ou d'agent gonflant et peu soluble, de 0 à 80 % d'agent gonflant et soluble ou d'agent gélifiable et soluble, de 0 à 50 % d'excipient permettant de réaliser des inclusions de principe actif, de 0 à 25 % d'agent adjuvant soluble dans l'eau agissant comme un agent à caractère hydrophile, de 0 à 50 % d'agent adjuvant insoluble dans l'eau agissant comme un agent à caractère hydrophobe.

10. Comprimés selon l'une quelconque des revendications 2 à 9, **caractérisés en ce que** les agents gonflants et insolubles ou les agents gonflants et peu solubles en présence de fluide biologique sont avantageusement choisis parmi le groupe des éthers de cellulose comme la carboxyméthylcellulose sodique, l'hydroxypropylcellulose réticulée, l'hydroxypropyl-cellulose de haut poids moléculaire, le groupe des esters de cellulose entériques et non entériques, les amidons modifiés comme le carboxyméthyl amidon, le copolymère de divinylbenzène/méthacrylate de potassium, le groupe des dérivés de l'acide méthacrylique comme les polyméthylméthacrylates, le groupe des crospovidones/ crospolyvidones, l'alcool polyvinylique de haut poids moléculaire, le groupe de l'acide alginique et de ses dérivés, le groupe des dérivés de l'acide acrylique comme l'acide acrylique réticulé avec du divinylglycol et son sel de calcium, le groupe des carraghénanes et de leurs dérivés, le copolymère de vinylacétate et de l'acide crotonique.

11. Comprimés selon l'une quelconque des revendications 2 à 10, **caractérisés en ce que** les agents gonflants et solubles ou les agents gélifiables et solubles en présence de fluide biologique convenant pour la mise en oeuvre de l'invention sont choisis parmi le groupe des éthers de cellulose comme la méthylcellulose, la carboxyméthylcellulose sodique, l'hydroxypropylmé-thylcellulose de faible poids moléculaire, le groupe des esters de cellulose entériques et non entériques, l'alcool polyvinylique de faible poids moléculaire, l'alcool polyvinylique de moyenne viscosité, le polyoxyéthylèneglycol, le groupe des povidones / polyvidones /copolyvidones, les scléroglucannes, les amidons et les amidons modifiés comme les amidons prégélatinisés, le groupe des carraghénanes et de leurs dérivés, le groupe de l'acide alginique et de ses dérivés.

12. Comprimés selon la revendication 2, **caractérisés en ce que** les excipients, utilisés en association avec le matériau bioadhésif, peuvent améliorer la bioadhésion comme la gomme guar, la gomme xanthane, le caroube, les carraghénates, la pectine, une protéine biologique ou synthétique utilisée seule ou en association avec d'autres protéines d'origine biologique ou synthétique, les cyclodextrines ou dérivés tels que les bétacyclodextrines, les hydroxypropylbétacyclodextrines, les bétacyclodextrines partiellement méthylées, les dérivés de l'acide acrylique, comme l'acide acrylique réticulé avec du divinylglycol et son sel de calcium.

13. Comprimés selon l'une quelconque des revendications 2 à 12, **caractérisés en ce que** les adjuvants solubles dans l'eau ou agissant comme des agents à caractère hydrophile sont choisis parmi le lactose, le mannitol, la silice colloïdale, les excipients du groupe des hydrocelluloses comme la cellulose microcristalline, les excipients dans le groupe des éthers de cellulose, la gélatine, les polyéthylèneglycols (PEG), les poloxamers et les pyrrolidones.

14. Comprimés selon l'une quelconque des revendications 2 à 13, **caractérisés en ce que** les adjuvants insolubles dans l'eau ou agissant comme des agents à caractère hydrophobe sont notamment choisis parmi l'huile de ricin hydrogénée, le stéarate de magnésium, les huiles naturelles et synthétiques, les cires naturelles ou semi-synthétiques, les esters d'acides gras et de polyoxyéthylène, les acides gras et les esters d'acides gras (mono et triglycérides) et leurs dérivés comme les acides gras polyéthoxylés (PEG-stéarate), les alcools gras et esters d'alcool gras et leurs dérivés comme les alcools gras polyéthoxylés (octyldodeceth-25) et le polychlorure de vinyle.

15. Comprimés selon l'une quelconque des revendications 1 à 14, **caractérisés en ce qu'**ils ont 2 à 30 mm de largeur environ et 1 à 8 mm d'épaisseur environ.

16. Comprimés selon l'une quelconque des revendications 1 à 15, **caractérisés en ce qu'**ils renferment des minigranules, des micropcapsules, des nanoparticules ou des nanocapsules enrobées ou non.

17. Procédé de fabrication des comprimés selon l'une quelconque des revendications 1 à 16, **caractérisé en ce qu'**il comprend
- la compression directe du mélange d'ingrédients élaboré pour former la couche bioadhésive,
- la compression directe du mélange d'ingrédients élaboré pour constituer la couche de principe actif et/ou de celui élaboré pour constituer la couche barrière, ou la compression de l'un de ces mélanges ou des deux mélanges tels qu'obtenus par granulation par voie humide.

## Patentansprüche

1. Bioadhäsive Tabletten, **dadurch gekennzeichnet, daß** sie in Form einer dreischichtigen Verbindung vorliegen, für eine im Wesentlichen die Schleimhaut durchdringende Verwendung hergestellt worden sind und die innere Schicht, die den größten Teil der Gesamtmenge des Wirkstoffs enthält,
- auf den einander gegenüberliegenden Flächen mit
• einer bioadhäsiven Schicht, welche die Gesamtmenge des bioadhäsiven Materials, aber keinen Wirkstoff umfasst, bei der Herstellung der Tablette direkt komprimierbar und in der Lage ist, durch Imprägnieren mit Wasser oder einer biologischen Flüssigkeit, die in der Umgebung der Schleimhaut vorhanden ist, an dieser anzuhaften, und die gewünschte Freisetzung des Wirkstoffs erlaubt, bzw.
• einer Schicht, die eine Barriere gegen die Diffusion des Wirkstoffs und das Eindringen von Wasser oder der biologischen Flüssigkeit bildet, und
auf den Seiten entweder mit der bioadhäsiven Schicht oder der Barriereschicht oder auch auf den beiden Flächen mit der bioadhäsiven Schicht und auf den Seiten mit der Barriereschicht bedeckt ist.

2. Tabletten nach Anspruch 1, **dadurch gekennzeichnet, daß** die bioadhäsive Schicht im Gemisch mit dem bioadhäsiven Material wenigstens ein quellendes und unlösliches Mittel oder ein quellendes und in Gegenwart der biologischen Flüssigkeit wenig lösliches Mittel und/oder ein quellendes und lösliches Mittel oder ein gelierbares und in Gegenwart der biologischen Flüssigkeit lösliches Mittel enthält, sowie gegebenenfalls wenigstens einen Exzipienten enthält, der eine Verbesserung der Bioadhäsion ermöglicht, und/oder ein Hilfsmittel enthält, das in Wasser löslich ist oder als ein Mittel mit hydrophilem Charakter wirkt.

3. Tabletten nach einem der vorstehenden Ansprüche, **dadurch gekennzeichnet, daß** das bioadhäsive Material im wesentlichen aus einem Polymer besteht, das durch Maleinsäureanhydrid oder durch ein Derivat wie Säure, Ester oder pharmazeutisch annehmbares Salz modifiziert ist.

4. Tabletten nach Anspruch 3, **dadurch gekennzeichnet, daß** das bioadhäsive Material im wesentlichen aus einem Copolymer aus Methylvinylether und Maleinsäureanhydrid besteht.

5. Tabletten nach Anspruch 2 oder 4, **dadurch gekennzeichnet, daß** die bioadbäsive Schicht bevorzugt 5 bis 100 % des bioadhäsiven Materials, 0 bis 80 % des quellenden und unlöslichen Mittels oder des quellenden und wenig löslichen Mittels, 0 bis 50 e des quellenden und löslichen Mittels oder des gelierbaren und löslichen Mittels, 0 bis 50 s des Exzipienten, der bei gemeinsamer Verwendung mit dem bioadhäsiven Polymer die Bioadhäsion verbessern kann, und 0 bis 80 % des in Wasser löslichen, als ein Mittel mit hydrophilem Charakter wirkenden Hilfsmittels enthält.

6. Tabletten nach einem der vorstehenden Ansprüche, **dadurch gekennzeichnet, daß** die Hauptschicht in vorteilhafter Weise im Gemisch mit dem Wirkstoff oder mit den Wirkstoffen wenigstens ein quellendes und unlösliches Mittel oder ein quellendes und in Gegenwart der biologischen Flüssigkeit wenig lösliches Mittel und/oder wenigstens ein quellendes und lösliches Mittel oder ein in Gegenwart der biologischen Flüssigkeit gelierbares und lösliches Mittel enthält, sowie gegebenenfalls wenigstens einen, die Aufnahme des Wirkstoffes ermöglichenden Exzipienten und/oder wenigstens einen in Wasser löslichen oder als ein Mittel mit hydrophilem Charakter wirkenden Exzipienten oder wenigstens einen in Wasser unlöslichen oder als ein Mittel mit hydrophobem Charakter wirkenden Exzipienten enthält.

7. Tabletten nach Anspruch 6, **dadurch gekennzeichnet, daß** die Hauptschicht vorteilhaft 70 bis 100 % der Gesamtmenge der Tablette an Wirkstoff, 0 bis 50 des quellenden und unlöslichen Mittels oder des quellenden und wenig löslichen Mittels, 0 bis 50 des quellenden und löslichen Mitteis oder des gelierbaren und löslichen Mittels, 0 bis 50 °-. des die Aufnahme des Wirkstoffes gestatten den Exzipienten, 0 bis 50 % des in Wasser löslichen, als Mittel mit hydrophilem Charakter wirkenden Hilfsmittels und 0 bis 50 des in Wasser unlöslichen Hilfsmittels, das als ein Mittel mit hydrophobem Charakter wirkt, enthält.

8. Tabletten nach einem der Ansprüche 1 bis 7, **dadurch gekennzeichnet, daß** die Barriereschicht im wesentlichen aus dem Wirkstoff in einem Anteil, der von 0 bis 30 ü der Gesamtmenge betragen kann, aus wenigstens einem quellenden und unlöslichen Mittel oder einem quellenden und in Gegenwart der biologischen Flüssigkeit wenig löslichen Mittel und/oder einem quellenden und löslichen Mittel oder einem in Gegenwart der biologischen Flüssigkeit gelierbaren und löslichen Mittel sowie im gegebenenen Falle aus wenigstens einem die Aufnahme des Wirkstoffes ermöglichenden Exzipienten und/oder einem in Wasser löslichen oder als ein Mittel mit hydrophilem Charakter wirkenden Hilfsmittel und/oder wenigstens einem in Wasser unlöslichen oder als ein Mittel mit hydrophobem Charakter wirkenden Mittel besteht.

9. Tablette nach Anspruch 8, **dadurch gekennzeichnet, daß** die Sperrschicht in vorteilhafter Weise 0 bis 30 % der Gesamtmenge der Tablette an Wirkstoff, 0 bis 80 °-. des quellenden und unlöslichen Mittels oder des quellenden und wenig löslichen Mittels, 0 bis 80 % des quellenden und löslichen Mittels oder des gelierbaren und löslichen Mittels, 0 bis 50 % des die Aufnahme des Wirkstoffes ermöglichenden Exzipienten, 0 bis 25 0- des in Wasser löslichen, als Mittel mit hydrophilem Charakter wirkenden Hilfsmittels und 0 bis 50 des in Wasser unlöslichen, als ein Mittel mit hydrophobem Charakter wirkenden Hilfsmittels einschließt

10. Tabletten nach einem der Ansprüche 2 bis 9, **dadurch gekennzeichnet, daß** die quellenden und unlöslichen Mittel oder die quellenden und in Gegenwart der biologischen Flüssigkeit wenig löslichen Mittel in vorteilhafter Weise aus der Gruppe der Zelluloseether, wie Natriumcarboxymethylzellulose, vernetzter Hydroxypropylzellulose, hochmolekularer Hydroxypropylzellulose, der enteralen und nichtenteralen Zelluloseester, der modifizierten Stärken, wie Carboxymethylstärke, Divinylbenzol/Kaliummethacrylatcopolymer, der Gruppe der Methacrylsäurederivate, wie Polymethylmethacrylate, der Gruppe der Crospovidone/Crospolyvidone, hochmolekularem Polyvinylalkohol, der Gruppe der Alginsäure und ihrer Derivate, der Gruppe der Acrylsäurederivate wie mit Divinylglykol vemetzter Acrylsäure und ihres Calciumsalzes, der Gruppe der Carraghenane und ihrer Derivate und dem Vinylacetat-Crotonsäurecopolymer ausgewählt sind.

11. Tabletten nach einem der Ansprüche 2 bis 10, **dadurch gekennzeichnet, daß** die quellenden und löslichen Mittel oder die gelierbaren und in Gegenwart der biologischen Flüssigkeit löslichen Mittel, die sich zur Ausführung der Erfindung eignen, unter der Gruppe der Zelluloseether wie Methylzellulose, Natriumcarboxymethylzellulose, niedrigmolekulare Hydroxypröpylmethylzellulose, der Gruppe der enteralen und nichtenteralen Zelluloseester, niedrigmolekularem Polyvinylalkohol, Polyvinylalkohol mit mittlerer Viskosität, Polyoxyethylenglycol, der Gruppe der Povidone/Polyvidone/Copolyvidone, den Skleroglucanen, den Stärken und den modifizierten Stärken, wie vorgelatinisierte Stärken, der Gruppe der Carraghenane und ihrer Derivate und der Gruppe der Alginsäure und ihrer Derivate ausgewählt sind.

12. Tabletten nach Anspruch 2, **dadurch gekennzeichnet, daß** die zusammen mit dem bioadhäsiven Material verwendeten Exzipienten die Bioadhäsion verbessern können, wie Guargummi, Xanthangummi, Johannisbrot, Carraghenate, Pectin, ein biologisches oder synthetisches Protein, das allein oder in Verbindung mit anderen Proteinen biologischen oder synthetischen Ursprungs verwendet wird, Cyclodextrine oder Derivate, wie Betacyclodextrine, Hydroxypropylbetacyclodextrine, partiell methylierte Betacyclodextrine, Acrylsäurederivate, wie mit Divinylglycol vernetzte Acrylsäure und ihr Calciumsalz.

13. Tabletten nach einem der Ansprüche 2 bis 12, **dadurch gekennzeichnet, daß** die in Wasser löslichen oder als Mittel mit hydrophilem Charakter wirkende Hilfsmittel unter Lactose, Mannit, kolloidaler Kieselsäure, Exzipienten aus der Gruppe der Hydrozellulosen wie mikrokristalline Zellulose, Exzipienten aus der Gruppe der Zelluloseether, Gelatine, Polyethylenglycolen (PEG), Poloxameren und Pyrrolidonen ausgewählt sind.

14. Tabletten nach einem der Ansprüche 2 bis 13, **dadurch gekennzeichnet, daß** die in Wasser unlöslichen oder als Mittel mit hydrophobem Charakter wirkenden Hilfsmittel insbesondere unter hydriertem Rizinusöl, Magnesiumstearat, natürlichen und synthetischen Ölen, natürlichen oder halbsynthetischen Wachsen, Estern von Fettsäuren mit Polyoxyethylen, Fettsäuren und Estern von Fettsäuren (Monound Triglyceride) und ihren Derivaten, wie polyethoxylierten Fettsäuren (PEG-Stearat), Fettalkoholen und Estern von Fettalkoholen und ihren Derivaten wie polyethoxylierten Fettalkoholen (Octyldodeceth-25) und Polyvinylchlorid ausgewählt sind.

15. Tabletten nach einem der Ansprüche 1 bis 14, **dadurch gekennzeichnet, daß** sie eine Länge von etwa 2 bis 30 mm und eine Di cke von etwa 1, bis 8 mm aufweisen.

16. Tabletten nach einem der Ansprüche 1 bis 15, **dadurch gekennzeichnet, daß** sie gegebenenfalls umhüllte Minigranulate, Mikrokapseln, Nanopartikel oder Nanokapseln enthalten.

17. Verfahren zur Herstellung von Tabletten nach einem der Ansprüche 1 bis 16, **dadurch gekennzeichnet, daß** es
- das direkte Komprimieren des zur Ausbildung der bioadhäsiven Schicht erarbeiteten Komponentengemisches und
- das direkte Komprimieren des zur Ausbildung der Wirkstoffschicht erarbeiteten Komponentengemisches und/oder des zur Ausbildung der Barriereschicht erarbeiteten Gemisches oder das Komprimieren eines dieser Gemische oder der beiden Gemische umfaßt, die durch Feuchtgranulation erhalten worden sind.

## Claims

1. Bioadhesive tablets, **characterized in that** they take the form of a triple-layer compound, developed for essentially transmucous use, in which the internal layer, comprising most of the total charge of active principle is covered:
- on its two opposing faces, respectively:
- with a bioadhesive layer, containing the total charge of bioadhesive material and not comprising active principle, said layer being directly compressible during the production of the tablet, and being capable of adhering to a mucous membrane by impregnation with water or with the biological fluid present in the environment of the mucous membrane and permitting the release, as desired, of active principle, and
- with a layer forming a barrier to diffusion of active principle and to the penetration of water or of said biological fluid
- and on the sides either by said bioadhesive layer, or by said barrier layer
or else it is covered on the two faces by the bioadhesive layers and on the sides by the barrier layer.

2. Tablets according to claim 1, **characterized in that** the bioadhesive layer contains, in a mixture with the bioadhesive material, at least one swelling agent which is insoluble or a swelling agent which is sparingly soluble in the presence of biological fluid, and/or a swelling agent which is soluble or a gelling agent which is soluble in the presence of biological fluid, with, if need be, at least one excipient which makes it possible to improve the bioadhesion and /or an adjuvant which is soluble in water or acts as an agent with a hydrophilic nature.

3. Tablets according to any one of the preceding claims, **characterized in that** the bioadhesive material is essentially composed of a polymer modified by maleic anhydride or by a derivative such as a pharmaceutically acceptable acid, ester or salt.

4. Tablets according to claim 3, **characterized in that** the bioadhesive material is essentially composed of a copolymer of methylvinylether and maleic anhydride.

5. Tablets according to claim 2 or 4, **characterized in that** the bioadhesive layer contains advantageously 5 to 100% of bioadhesive material, 0 to 80% of insoluble swelling agent or sparingly soluble swelling agent, 0 to 50% of soluble swelling agent or soluble gelling agent, 0 to 50% of excipient which, used in combination with the bioadhesive polymer, may improve bioadhesion, and 0 to 80% of adjuvant which is soluble in water acting as an agent with a hydrophilic nature.

6. Tablets according to any one of the preceding claims, **characterized in that** the main layer contains advantageously, in a mixture with the active principle(s), at lest one swelling agent which is insoluble or a swelling agent which is sparingly soluble in the presence of biological fluid, and/or at least one swelling agent which is soluble or a gelling agent which is soluble in the presence of biological fluid with, if need be, at least one excipient which makes it possible to obtain inclusions of active principle and/or at least one excipient which is soluble in water or acts as an agent with a hydrophilic nature, or at least one excipient which is insoluble in water or acts as an agent with a hydrophobic nature.

7. Tablets according to claim 6, **characterized in that** the main layer contains advantageously 70 to 100% of the total charge of active principle of the tablet, 0 to 50% of insoluble swelling agent or sparingly soluble swelling agent, 0 to 50% of soluble swelling agent or soluble gelling agent, 0 to 50% of excipient which makes it possible to obtain inclusions of active principle, 0 to 50% of adjuvant which is soluble in water acting as an agent with a hydrophilic nature, 0 to 50% of adjuvant which is insoluble in water acting as an agent with a hydrophobic nature.

8. Tablets according to any one of claims 1 to 7 **characterized in that** the barrier layer is essentially formed from the active principle in a proportion which may range from 0 to 30% of the total charge, at least one swelling agent which is insoluble or a swelling agent which is sparingly soluble in the presence of biological fluid and/or a swelling agent which is soluble or a gelling agent which is soluble in the presence of biological fluid with, if need be, at least one excipient which makes it possible to obtain inclusions of active principle, and/or an adjuvant which is soluble in water or acts as an agent with a hydrophilic nature and/or at least one agent which is insoluble in water or acts as an agent with a hydrophobic nature.

9. Tablets according to claim 8, **characterized in that** the barrier layer contains advantageously 0 to 30% of the total charge of active principle of the tablet, 0 to 80% of insoluble swelling agent or sparingly soluble swelling agent, 0 to 80% of soluble swelling agent or soluble gelling agent, 0 to 50% of excipient which makes it possible to obtain inclusions of active principle, 0 to 25% of adjuvant which is soluble in water acting as an agent with a hydrophilic nature, 0 to 50% of adjuvant which is insoluble in water acting as an agent with a hydrophobic nature.

10. Tablets according to any one of claims 2 to 9, **characterized in that** the swelling agents which are insoluble or the swelling agents which are sparingly soluble in the presence of biological fluid are advantageously chosen from the group of cellulose ethers such as sodium carboxymethylcellulose, cross-linked hydroxypropylcellulose, high molecular weight hydroxypropylcellulose, the group of enteric and non-enteric cellulose esters, modified starches such as carboxymethyl starch, the copolymer of divinylbenzene/potassium methacrylate, the group of derivatives of methacrylic acid such as polymethylmethacrylates, the group of crospovidones/crospolyvidones, high molecular weight polyvinyl alcohol, the group of alginic acid and derivatives thereof, the group of derivatives of acrylic acid such as cross-linked acrylic acid with divinylglycol and its calcium salt, the group of carrageenans and derivatives thereof, the copolymer of vinyl acetate and crotonic acid.

11. Tablets according to any one of claims 2 to 10, **characterized in that** the swelling agents which are soluble or the gelling agents which are soluble in the presence of biological fluid suitable for the implementation of the invention are chosen from the group of cellulose ethers such as methylcellulose, sodium carboxymethylcellulose, low molecular weight hydroxypropylmethylcellulose, the group of enteric and non-enteric cellulose esters, low molecular weight polyvinyl alcohol, medium viscosity polyvinyl alcohol, polyoxyethylene glycol, the group of povidones/polyvidones/copolyvidones, scleroglucanes, starches and modified starches such as pregelatinized starches, the group of carrageenates and derivatives thereof, the group of alginic acid and derivatives thereof.

12. Tablets according to claim 2, **characterised in that** the excipients used in combination with the bioadhesive material may improve bioadhesion such as guar gum, xanthan gum, carob, carrageenates, pectin, a biological or synthetic protein used alone or in combination with other proteins of biological or synthetic origin, cyclodextrins or derivatives such as betacyclodextrins, hydroxypropyl betacyclodextrins, partially methylated betacyclodextrins, derivatives of acrylic acid such as cross-linked acrylic acid with divinylglycol and its calcium salt.

13. Tablets according to any one of claims 2 to 12, **characterized in that** the adjuvants which are soluble in water or act as agents with a hydrophilic nature are chosen from lactose, mannitol, colloidal silica, excipients of the group of hydrocelluloses such as microcrystalline cellulose, excipients in the group of cellulose ethers, gelatin, polyethylene glycols (PEG), poloxamers and pyrrolidones.

14. Tablets according to any one of claims 2 to 13, **characterized in that** the adjuvants which are insoluble in water or act as agents with a hydrophobic nature are chosen in particular from hydrogenated castor oil, magnesium stearate, natural and synthetic oils, natural or semisynthetic waxes, esters of fatty acids and poloxyethylene, fatty acids and esters of fatty acids (mono and triglycerides) and derivatives thereof such as polyethoxylated fatty acids (PEG stearate,) fatty alcohols and esters of fatty alcohol and derivatives thereof such as polyethoxylated fatty alcohol (octyldodeceth-25) and polyvinyl chloride.

15. Tablets according to any one of claims 1 to 14, **characterized in that** they are about 2 to 30 mm wide and about 1 to 8 mm thick.

16. Tablets according to any one of claims 1 to 15, **characterized in that** they contain coated or uncoated minigranules, microcapsules, nanoparticles or nanocapsules.

17. Process for the production of tablets according to any one of claims 1 to 16, **characterized in that** it comprises
- the direct compression of the mixture of ingredients produced to form the bioadhesive layer,
- the direct compression of the mixture of ingredients produced to constitute the layer of active principle and/or of the mixture of ingredients produced to constitute the barrier layer, or the compression of one of these mixtures or of the two mixtures as obtained by wet granulation.
